(19) Europäisches Patentamt
European Patent Office
Office européen des brevets

(11) Veröffentlichungsnummer: **0 332 007 B1**

(12) **EUROPÄISCHE PATENTSCHRIFT**

(45) Veröffentlichungstag der Patentschrift: **25.11.92**

(21) Anmeldenummer: **89103414.2**

(22) Anmeldetag: **27.02.89**

Verbunden mit 89902770.0/0373186 (europäische Anmeldenummer/Veröffentlichungsnummer) durch Entscheidung vom 05.12.91.

(51) Int. Cl.[5]: **C07C 69/75**, C07C 69/753, C07C 69/76, C07C 69/92, C07D 239/26, C07C 43/225, C09K 19/30, C09K 19/20, C09K 19/34

# (54) Derivate des 2,3-Difluorhydrochinons.

(30) Priorität: **10.03.88 DE 3807801**

(43) Veröffentlichungstag der Anmeldung:
**13.09.89 Patentblatt 89/37**

(45) Bekanntmachung des Hinweises auf die Patenterteilung:
**25.11.92 Patentblatt 92/48**

(84) Benannte Vertragsstaaten:
**AT BE CH DE ES FR GB GR IT LI LU NL SE**

(56) Entgegenhaltungen:
**EP-A- 0 023 728**
**EP-A- 0 051 738**
**WO-A-88/09322**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Reiffenrath, Volker**
**Jahnstrasse 18**
**W-6101 Rossdorf(DE)**
Erfinder: **Krause, Joachim, Dr.**
**Samuel-Morse-Strasse 14**
**W-6110 Dieburg(DE)**
Erfinder: **Wächtler, Andreas, Dr.**
**Goethestrasse 34**
**W-6103 Griesheim(DE)**
Erfinder: **Weber, Georg**
**Wilhelm-Leuschner-Strasse 38**
**W-6106 Erzhausen(DE)**
Erfinder: **Geelhaar, Thomas, Dr.**
**Trajanstrasse 12**
**W-6500 Mainz(DE)**
Erfinder: **Coates, David, Dr.**
**87, Sopwith Crescent Merley**
**Wimborne Dorset BH21 3SW(GB)**
Erfinder: **Sage, Ian Charles, Dr.**
**58, Wentworth Drive**
**Broadstone Dorset BH18 8EG(GB)**
Erfinder: **Greenfield, Simon, Dr.**
**2, Blackbird Close**
**Creekmoor Poole, BH17 7YA(GB)**

EP 0 332 007 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Derivate des 2,3-Difluorhydrochinons des Formel I,

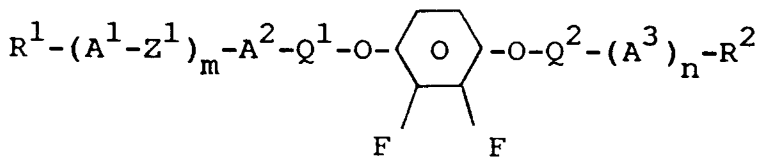

I

worin

$R^1$ und $R^2$ jeweils unabhängig voneinander unsubstituiertes, einfach durch Cyan oder mindestens einfach durch Fluor oder Chlor substituiertes Alkyl mit 1 bis 15 C-Atomen oder Alkenyl mit 3 bis 15 C-Atomen, wobei in diesen Resten auch eine $CH_2$-Gruppe durch -O-, -CO-, -CO-O-, -O-CO- oder -O-CO-O-ersetzt sein kann,

$A^1$, $A^2$ und $A^3$ jeweils unabhängig voneinander

(a) trans-1,4-Cyclohexylen, worin auch eine oder zwei nicht benachbarte $CH_2$-Gruppen durch -O- und/oder -S- ersetzt sein können,

(b) 1,4-Phenylen, worin auch eine oder zwei CH-Gruppen durch N ersetzt sein können,

(c) Rest aus der Gruppe bestehend aus Piperidin-1,4-diyl, 1,4-Bicyclo(2,2,2)octylen, 1,3,4-Thiadiazol-2,5-diyl, Naphtalin-2,6-diyl, Decahydronaphtalin-2,6-diyl und 1,2,3,4-Tetrahydronaphtalin-2,6-diyl,

wobei die Reste (a) und (b) durch F, Cl, $CH_3$ oder CN einoder mehrfach substituiert sein können,

$Z^1$ -CO-O-, -O-CO-, $-CH_2-CH_2-$, $-OCH_2-$, $-CH_2O-$, -C≡C-oder eine Einfachbindung,

$Q^1$ und $Q^2$ jeweils unabhängig voneinander -CO- oder $-CH_2-$,

m und n jeweils 0, 1 oder 2, und

(m + n) 0, 1 oder 2 bedeutet.

Der Einfachheit halber bedeuten im folgenden Cyc eine 1,4-Cyclohexylengruppe und $PheF_2$ bedeutet eine Gruppe der Formel

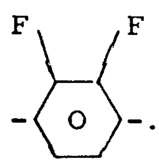

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Die Verbindungen der Formel I zeichnen sich durch eine deutlich negative Anisotropie der Dielektrizitätskonstante aus und werden in einem elektrischen Feld mit ihren Moleküllängsachsen senkrecht zur Feldrichtung ausgerichtet.

Dieser Effekt ist bekannt und wird zur Steuerung der optischen Transparenz in verschiedenen Flüssigkristallanzeigen ausgenützt, so z.B. in Flüssigkristallzellen vom Lichtstreuungstyp (dynamische Streuung) von sogenannten DAP-Typ (Deformation aufgerichteter Phasen) oder ECB-Typ (electrically controlled birefringence) oder vom Gast/Wirt-Typ (guest-host interaction).

Die Verbindungen der Formel I eignen sich auch als Komponenten von nematischen oder chiral nematischen flüssigkristallinen Medien mit einer insgesamt positiven dielektrischen Anisotropie. Die Verbindungen der Formel I dienen hier zur Erhöhung von $\epsilon_{\perp}$. Die negative dielektrische Anisotropie der erfindungsgemäßen Verbindungen wird hier durch Zusatz dielektrisch stark positiver Komponenten überkompensiert. Derartige Medien werden für TN-Displays mit 90°-Verdrillung oder insbesondere für höherverdrillte TN-Zellen verwendet, da sie dort zu besonders steilen Kennlinien führen.

Weiterhin eignen sich Verbindungen der Formel I als Komponenten chiraler getilteter smektischer Medien. Chirale getiltete smektische flüssigkristalline Medien mit ferroelektrischen Eigenschaften können hergestellt werden, indem man Basis-Mischungen mit einer oder mehreren getilteten smektischen Medien

mit einem geeigneten chiralen Dotierstoff versetzt (L. A. Veresnev et al., Mol. Cryst. Liq. Cryst. 89, 327 (1982); H.R. Brand et al., J. Physique 44 (lett.), L-771 (1983). Solche Medien können als Dielektrika für schnell schaltende Displays verwendet werden, die auf dem von Clark und Lagerwall beschriebenen Prinzip der SSFLC-Technologie (N. A. Clark und S.T. Lagerwall, Appl. Phys. Lett. 36, 899 (1980); USP 4,367,924) auf der Basis der ferroelektrischen Eigenschaften des chiralen getilteten Mediums beruhen.

Es sin bisher bereits eine Reihe von flüssigkristallinen Verbindungen mit schwach negativer dielektrischer Anisotropie synthetisiert worden. Hingegen sind noch relativ wenig Flüssigkristallkomponenten mit großer negativer Anisotropie der Dielektrizitätskonstante bekannt. Zudem weisen letztere im allgemeinen Nachteile auf, wie z.B. schlechte Löslichkeit in Mischungen, hohe Viskosität, hohe Schmelzpunkte und chemische Instabilität. Es besteht daher ein Bedarf an weiteren Verbindungen mit negativer dielektrischer Anisotropie, die es erlauben, die Eigenschaften von Mischungen für verschiedenste elektro-optische Anwendungen weiter zu verbessern.

Flüssigkristallverbindungen mit negativer dielektrischer Anisotropie, welche zwei oder drei über Carboxylgruppen oder Kovalenzbindung verknüpfte Ringe und eine oder mehrere seitliche Gruppen, wie Halogen, Cyano oder Nitrogruppen aufweisen, sind bekannt aus DE 22 40 864, DE 26 13 293, DE 28 35 662, DE 28 36 086 und EP 023 728.

In der EP 084 194 werden in einer breiten Formel die hier beanspruchten Verbindungen umfaßt. Jedoch sind dort keine Einzelverbindungen der erfindungsgemäßen Formel genannt. Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend günstig gelegene Mesophasenbereiche aufweisen und sich durch eine große negative Anisotropie der Dielektrizität bei gleichzeitiger niedriger Viskosität auszeichnen.

Auch fehlt dort jeglicher Hinweis auf die Möglichkeit des Einsatzes der erfindungsgemäßen Verbindungen in Displays, die auf der SSFLC-Technologie beruhen, da die dort beanspruchten Verbindungen geringe smektische Tendenzen aufweisen.

Weiterhin sind Dibenzoesäureester des 2,3-Dichlorhydrochinons bekannt (z.B Bristol et al., J. Org. Chem. 39, 3138 (1974) oder Clanderman et al., J. Am. Chem. Soc. 97, 1585 (1975)), die allerdings monotrop sind bzw. sehr kleine Mesophasenbereiche aufweisen. Die von Eidenschink et al. beschriebenen Ester der 4-hydroxy-2,3-dichlorbenzoesaüre (Angew. Chem. 89, 103 (1977)) besitzen ebenfalls nur schmale Mesophasenbereiche.

Die aus der DE OS 29 33 563 bekannten 4-Alkyl-2,3-dichlorphenyl-4′-alkylbicyclohexyl-4-carbonsäureester erlauben wegen ihrer hohen Viskosität keine technische Anwendung.

Der Erfindung lag die Aufgabe zugrunde stabile, flüssigkristalline oder mesogene Verbindungen mit einer großen negativen Anisotropie der Dielektrizität und gleichzeitig geringer Viskosität aufzuzeigen.

Es wurde gefunden, daß die Verbindungen der Formel I als Komponenten flüssigkristalliner Medien vorzüglich geeignet sind. Insbesondere sind mit ihrer Hilfe stabile flüssigkristalline Medien mit breitem Mesophasenbereich und vergleichsweise niedriger Viskosität herstellbar.

Weiterhin eignen sich die Verbindungen der Formel I als Komponenten chiraler getilteter smektischer flüssigkristalliner Medien.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Medien eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/ oder den Pitch eines solchen Dielektrikums zu varriieren.

Die Verbindungen der Formel I eignen sich ferner als Zwischenprodukte zur Herstellung anderer Substanzen, die sich als Bestandteile flüssigkristalliner Medien verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine entsprechende Carbonsäure bzw. Hydroxyverbindung oder eines ihrer reaktions-fähigen Derivate mit einer entsprechenden Hydroxyverbindung oder einem seiner reaktionsfähigen Derivate umsetzt.

Weiterhin ist Gegenstand der Erfindung die Verwendung der Verbindungen der Formel I als Komponenten flüssigkristalliner Medien. Gegenstand der Erfindung sind ferner flüssigkristalline Medien mit einem Gehalt an mindestens einer Verbindung der Formel I, sowie Flüssigkristall-Anzeigeelemente, die derartige Medien enthalten. Derartige Medien weisen besonders vorteilhafte elastische Konstanten auf, und eignen sich wegen ihrer niedrigen $\Delta\ \epsilon/\epsilon_{\perp}$, -Werte besonders für TFT-Mischungen.

Ferner sind Gegenstand der Erfindung alle neuen Zwischenprodukte zur Herstellung der erfindungsgemäßen Verbindungen.

Vor- und nachstehen haben $R^1$, $R^2$, $A^1$, $A^2$, $A^3$, $Z^1$, $Q^1$, $Q^2$, n und m die angegebene Bedeutung, sofern nicht ausdrücklich etwas anderes vermerkt ist.

Die Verbindungen der Formel I umfassen dementsprechend bevorzugte Verbindungen mit zwei Ringen der Teilformel Ia:

$R^1$-$A^2$-$Q^1$-O-Phe$F_2$-O-$Q^2$-$R^2$ Ia

bevorzugte Verbindungen mit drei Ringen der Teilformeln Ib bis Id:

$R^1$-$A^2$-$Q^1$-O-Phe$F_2$-O-$Q^2$-$A^3$-$R^2$ Ib
$R^1$-$A^1$-$A^2$-$Q^1$-O-Phe$F_2$-O-$Q^2$-$R^2$ Ic
$R^1$-$A^1$-$Z^1$-$A^2$-$Q^1$-O-Phe$F_2$-O-$Q^2$-$R^2$ Id

und bevorzugte Verbindungen mit vier Ringen der Teilformeln Ie und If:

$R^1$-$A^1$-$A^1$-$A^2$-$Q^1$-O-Phe$F_2$-O-$Q^2$-$R^2$ Ie
$R^1$-$A^1$-$A^2$-$Q^1$-O-Phe$F_2$-O-$Q^2$-$A^3$-$R^2$ If

Darunter sind diejenigen der Teilformeln Ia, Ib und Ic besonders bevorzugt.

In den Verbindungen der vor- und nachstehenden Formeln bedeuten $R^1$ und $R^2$ vorzugsweise Alkyl oder Alkoxy.

Weiterhin bevorzugt sind Verbindungen der vor- und nachstehenden Formeln, in denen einer der Reste $R^1$ und $R^2$, Alkenyl oder Oxaalkyl (z.B. Alkoxymethyl) bedeutet.

$R^1$ und $R^2$ weisen in den vor- und nachstehenden Formeln vorzugsweise 2-12 C-Atome, insbesondere 3-10 C-Atome auf. In $R^1$ und $R^2$ können auch eine oder zwei $CH_2$-Gruppen ersetzt sein. Vorzugsweise ist nur eine $CH_2$-Gruppe ersetzt durch -O-, oder -CH = CH-.

In den vor- und nachstehenden Formeln bedeuten $R^1$ und $R^2$ vorzugsweise Alkyl, Alkoxy oder eine andere Oxaalkylgruppe, ferner auch Alkylgruppen, in denen eine oder zwei $CH_2$-Gruppen durch -CH = CH- ersetzt sein können.

Falls $R^1$ und $R^2$ Alkylreste, in denen auch eine ("Alkoxy" bzw. "Oxaalkyl") oder zwei ("Alkoxylakoxy" bzw. "Dioxaalkyl") nicht benachbarte $CH_2$-Gruppen durch O-Atome ersetzt sein können, bedeuten, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxypropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4-, 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Falls $R^1$ und $R^2$ einen Alkylrest bedeuten, in dem eine $CH_2$-Gruppe durch -CH = CH- ersetzt ist, so ist die trans-Form bevorzugt. Dieser Alkenylrest kann geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls die Reste $R^1$ bzw. $R^2$ einfach durch Cyan oder mindestens einfach durch Fluor oder Chlor substituiert sind, handelt es sich vorzugsweise um $\omega$-Fluor- oder $\omega$-Cyan-n-Alkyl mit 1-12 C-Atomen, um n-Alkylgruppen mit 2-12 C-Atomen mit Substitution durch Fluor, Chlor oder Cyan an einem sekundären C-Atom oder um Perfluor-n-alkylgruppen mit 1-12 C-Atomen, worin auch eine oder mehrere $CF_2$-Gruppen durch -O- und/oder $-CH_2-$ ersetzt sein können.

Verbindungen der Formel I mit verzweigten Flügelgruppen $R^1$ und/oder $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste $R^1$ und/oder $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 4-Methylhexyl, 2-Nonyl, 2-Decyl, 2-Dodecyl, 6-Methyloctoxy.

Formel I umfaßt sowohl die Racemate dieser Verbindungen als auch die optischen Antipoden sowie deren Gemische.

$A^1$, $A^2$ und $A^3$ bedeuten jeweils unabhängig voneinander vorzugsweise trans-1,4-Cyclohexylen, 1,4-Phenylen, 2- oder 3-Fluor-1,4-phenylen, 2,3-Difluor-1,4-phenylen, Pyrimidin-2,5-diyl oder Pyridin-2,5-diyl. Vorzugsweise enthalten die erfindungsgemäßen Verbindungen nur eine 2-Fluor-1,4-phenylen-, 3-Fluor-1,4-phenylen, Pyridin-2,5-diyl- oder Pyrimidin-2,5-diyl-Gruppe. Besonders bevorzugte Bedeutungen für $A^1$, $A^2$ und $A^3$ sind trans-1,4-Cyclohexylen und 1,4-Phenylen.

m und n sind jeweils vorzugsweise 0 oder 1. $Z^1$ ist vorzugsweise $-CH_2CH_2-$ oder eine Einfachbindung. $Q^1$ ist im Falle $A^2$ = Rest (a) [d.h. beispielsweise 1,4-Cyclohexylen] -CO- oder $-CH_2-$, wobei $-CH_2-$ bevorzugt ist. Im Falle $A^2$ = Rest (b) [d.h. beispielsweise 1,4-Phenylen] ist $Q^1$ vorzugsweise -CO-.

Unter den Verbindungen der Formeln I sowie Ia bis If sind diejenigen bevorzugt, in denen mindestens einer der darin enthaltenen Reste eine der angegebenen bevorzugten Bedeutungen hat.

Besonders bevorzugt sind Derivate des 2,3-Difluorhydrochinons der Formel Ia′,

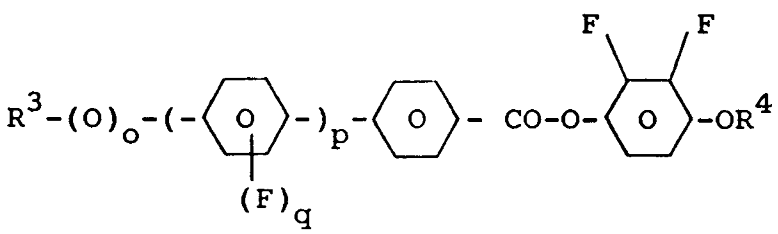

Ia'

worin

$R^3$ und $R^4$ jeweils unabhängig voneinander Alkyl mit 1 bis 15 C-Atomen, und o, p und q 0 oder 1 bedeutet, sowie

Derivate des 2,3-Difluorhydrochinons der Formel Ia′′

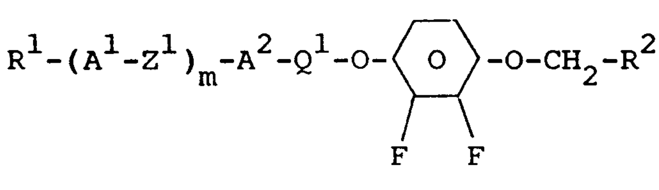

Ia''

worin $R^1$, $R^2$ und $Q^1$ die in Anspruch 1 angegebene Bedeutung haben, m 0 oder 1, $Z^1$ $-CH_2CH_2-$ oder eine Einfachbindung, und $A^1$ und $A^2$ jeweils trans-1,4-Cyclohexylen bedeuten.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Verbindungen der Formel I sind ausgehend von 1,2-Difluorbenzol zugänglich. Dieses wird nach bekanntem Verfahren (z.B. A.M. Roe et al., J. Chem. Soc. Chem. Comm., 22, 582 (1965)) metalliert und mit dem entsprechenden Elektrophil umgesetzt. Mit dem so erhaltenen 1-substituierten 2,3-Difluorbenzol läßt sich diese Reaktionssequenz ein zweites Mal z.B. mit tert.-Butylhydroperoxid als Elektrophil durchführen und man gelangt so zu den 1-substituierten 2,3-Difluor-phenolen. 1,2-Difluorbenzol bzw. 1-substituiertes 2,3-Difluorbenzol wird in einem inerten Lösungsmittel wie Diethylether, Tetrahydrofuran, Dimethoxyethan, tert-Butylmethylether oder Dioxan, Kohlenwasserstoffen wie Hexan, Heptan, Cyclohexan, Benzol oder Toluol oder Gemischen dieser Lösungsmittel gegebenenfalls unter Zusatz eine Komplexierungsmittels wie Tetramethylethylendiamin (TMEDA) oder Hexamethylphosphorsäuretriamid mit Phenyllithium, Lithiumtetramethylpiperidin, n-, sek- oder tert-Butyllithium bei Temperaturen von -100 °C bis +50 °C vorzugsweise -78 °C bis 0 °C umgesetzt.

Die Lithium-2,3-difluorphenyl-Verbindungen werden bei -100 °C bis 0 °C vorzugsweise bei -50 °C mit den entsprechenden Elektrophilen umgesetzt. Geeignete Elektrophile sind Aldehyde, Ketone, Nitrile, Epoxide, Carbonsäure-Derivate wie Ester, Anhydride oder Halogenide, Halogenameisensäureester oder Kohlendioxid.

Zur Umsetzung mit aliphatischen oder aromatischen Halogen-Verbindungen werden die Lithium-2,3-difluorphenyl-Verbindungen transmetalliert und unter Übergangsmetallkatalyse gekoppelt. besonders geeignet sind hierfür die Zink- (vgl. DE OS 36 32 410) oder die Titan-2,3-difluorphenyl-Verbindungen (vgl. DE OS 37 36 489).

Die Verbindungen der Formel I können hergestellt werden durch Veresterung entsprechender Carbonsäuren (oder ihrer reaktionsfähigen Derivate) mit Alkoholen bzw. Phenolen (oder ihren reaktionsfähigen Derivaten).

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäure-hexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 ° und +250°, vorzugsweise zwischen -20 ° und +80 °. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natrium-carbonat, Natriumhydrogencarbonat, Kaliumcarbonat oder Kaliumhydrogencarbonat, Alkalimetallacetate wie Natrium-oder Kaliumacetat, Erdalkalimetallhydroxide wie Calciumhydroxid oder organische Basen wie Triethylamin, Pyridin, Lutidin, Collidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung der Säurechlorids oder Anhydrids in Diethylether, Aceton oder DMF versetzt, zweckmäßig bei Temperaturen zwischen etwa -25 ° und +20°.

Die Methylether der Formel I können durch bekannte Veretherungen dargestellt werden, z.B. indem eine Verbindung der Formel $R^{-1}$-$(A^1$-$Z^1)_m$-$A^2$-$Q^1$-O-PheF$_2$-$Z_A$ bzw. $R^2$-$(A^3)_n$-$Q^2$-O-PheF$_2$-$Z_A$, worin $Z_A$ OH oder OMe, SH oder SMe bedeutet, wobei Me ein Äquivalent eines Metallkations und Hal ein Halogenatom, vorzugsweise Chlor oder Brom ist (reaktionsfähiges Derivat der Phenole), mit einer Verbindung der Formel

$R^2$-$(A^3)_n$-$Z_B$ bzw. $R^1$-$(A^1$-$Z^1)_m$-$A^2$-$Z_B$, worin $Z_B$ -$CH_2$-OH oder -$CH_2$-Hal (reaktionsfähiges Derivat) bedeutet, in Gegenwart einer Base umsetzt. Die Reste $R^1$, $R^2$, $A^1$, $A^2$, $A^3$, $Z^1$, m, n, $Q^1$ und $Q^2$ haben dabei die bei der Formel I angegebenen Bedeutungen. Die Reaktionsbedingungen für diese Ethersynthesen sind die für derartige Umsetzungen üblichen; als Lösungsmittel werden polare, aprotische verwendet, zum Beispiel Dimethylsulfoxid, N,N-Dimethylformamid oder N-Methylpyrrolidon; als Basen dienen vorzugsweise Alkalisalze schwacher Säuren, zum Beispiel Natriumacetat, Kaliumcarbonat oder Natriumcarbonat. Die Reaktionen können bei Temperaturen zwischen 0 °C und dem Siedepunkt der am niedrigsten siedenden Komponente des Reaktionsgemisches durchgeführt werden; es hat sich als besonders vorteilhaft erwiesen, Temperaturen zwischen 60 und 120 °C anzuwenden. Die Ausgangsmaterialien sind zum Teil bekannt, zum Teil können sie analog zu bekannten Verbindungen nach Standardverfahren der synthetischen organischen Chemie hergestellt werden.

Ein wichtiges neuen Zwischenprodukt zur Synthese von erfindungsgemäßen Verbindungen ist 2,3-Difluorhydrochinon. Diese neue Verbindung, die ebenfalls Gegenstand der Erfindung ist, kann durch Oxidation des bekannten 2,3-Difluorphenols in an sich bekannter Weise, z.B. durch Oxidation mit Kaliumperoxodisulfat in alkalischer, wäßriger Lösung erhalten werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4′-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R′-L-E-R″ 1
R′-L-COO-E-R″ 2
R′-L-OOC-E-R″ 3
R′-L-$CH_2CH_2$-E-R″ 4
R′-L-C≡C-E-R″ 5

In der Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R′ und R″ bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R′ und R″ voneinander verschieden, wobei einer dieser Reste meist

Alkyl oder Alkenyl ist. In den Verbindungen der Teilformeln 1b, 2b, 3b, 4b und 5b bedeutet R'' -CN, $-CF_3$, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:

Gruppe 1: 20 bis 90 %, insbesondere 30 bis 90 %,
Gruppe 2: 10 bis 80 %, insbesondere 10 bis 50 %,

wobei die Summe der Anteile der erfindungsgemäßen Verbindungen und der Verbindungen aus den Gruppen 1 und 2 bis zu 100% ergeben.

Die erfindungsgemäßen Medien enthalten vorzugsweise 1 bis 40 %, insbesondere vorzugsweise 5 bis 30% an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erflogt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie. Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

0,1 mol 2,3-Difluor-4-ethoxyphenol (herstellbar aus 2,3-Difluorphenol durch Alkylierung mit Diethylsulfat/Kaliumcarbonat in Dimethylformamid, Lithiierung bei -70 bis -80°, Umsetzung mit N-Methylpiperidin une Oxidation des Aldehyds nach Baeyer-Villiger mit Perameisensäure) und 0,1 mol Pyridin wurden in 100 ml Toluol gelöst. Bei 80° tropft man 0,1 mol trans-4-Pentylcyclohexancarbonsäurechlorid zu und rührt 3 Stunden nach. Das ausgefallene Pyridinhydrochlorid wird abgesaugt, das Toluol abdestilliert und das zurückbleibende (2,3-Difluor-4-ethoxyphenyl)-trans-4-pentylcyclohexanoat durch Kristallisation aus Ethanol gereinigt, F. 48°, K. 62,5°.

Analog werden hergestellt:

(2,3-Difluor-4-ethoxyphenyl)-trans-4-propylcyclohexanoat, F 50°, K 52°
(2,3-Difluor-4-ethoxyphenyl)-trans-4-ethylcyclohexanoat
(2,3-Difluor-4-ethoxyphenyl)-trans-4-butylcyclohexanoat, K 59° N (48°) I
(2,3-Difluor-4-ethoxyphenyl)-trans-4-heptylcyclohexanoat, K 53° N 66° I
(2,3-Difluor-4-methoxyphenyl)-trans-4-ethylcyclohexanoat
(2,3-Difluor-4-methoxyphenyl)-trans-4-propylcyclohexanoat
(2,3-Difluor-4-methoxyphenyl)-trans-4-butylcyclohexanoat
(2,3-Difluor-4-methoxyphenyl)-trans-4-pentylcyclohexanoat
(2,3-Difluor-4-methoxyphenyl)-trans-4-heptylcyclohexanoat
(2,3-Difluor-4-propoxyphenyl)-trans-4-ethylcyclohexanoat
(2,3-Difluor-4-propoxyphenyl)-trans-4-propylcyclohexanoat
(2,3-Difluor-4-propoxyphenyl)-trans-4-butylcyclohexanoat
(2,3-Difluor-4-propoxyphenyl)-trans-4-pentylcyclohexanoat

(2,3-Difluor-4-propoxyphenyl)-trans-4-heptylcyclohexanoat
(2,3-Difluor-4-butoxyphenyl)-trans-4-ethylcyclohexanoat
(2,3-Difluor-4-butoxyphenyl)-trans-4-propylcyclohexanoat
(2,3-Difluor-4-butoxyphenyl)-trans-4-butylcyclohexanoat
(2,3-Difluor-4-butoxyphenyl)-trans-4-pentylcyclohexanoat
(2,3-Difluor-4-butoxyphenyl)-trans-4-heptylcyclohexanoat
(2,3-Difluor-4-hexoxyphenyl)-trans-4-ethylcyclohexanoat
(2,3-Difluor-4-hexoxyphenyl)-trans-4-propylcyclohexanoat
(2,3-Difluor-4-hexoxyphenyl)-trans-4-butylcyclohexanoat
(2,3-Difluor-4-hexoxyphenyl)-trans-4-pentylcyclohexanoat
(2,3-Difluor-4-hexoxyphenyl)-trans-4-heptylcyclohexanoat
(2,3-Difluor-4-octoxyphenyl)-trans-4-ethylcyclohexanoat
(2,3-Difluor-4-octoxyphenyl)-trans-4-propylcyclohexanoat
(2,3-Difluor-4-octoxyphenyl)-trans-4-butylcyclohexanoat
(2,3-Difluor-4-octoxyphenyl)-trans-4-pentylcyclohexanoat
(2,3-Difluor-4-octoxyphenyl)-trans-4-heptylcyclohexanoat, K 43° N 64° I.

Beispiel 2

Analog Beispiel 1 erhält man aus demselben Phenol durch Umsetzung mit 4-(trans-4-Propylcyclohexyl)-cyclohexancarbonsäurechlorid (2,3-Difluor-4-ethoxyphenyl)-trans-4-(trans-4-propylcyclohexyl)cyclohexanoat, K 88° $S_A$ 100° N 223° I.

Analog werden hergestellt:
(2,3-Difluor-4-ethoxyphenyl)-trans-4-(trans-4-ethylcyclohexyl-cyclohexanoat
(2,3-Difluor-4-ethoxyphenyl)-trans-4-(trans-4-butylcyclohexyl)-cyclohexanoat, K 91° $S_B$ 109° $S_A$ 128° N 217° I
(2,3-Difluor-4-ethoxyphenyl)-trans-4-(trans-4-pentyl cyclohexyl)-cyclohexanoat, K 95° $S_B$ 119° $S_A$ 143° N 220° I
(2,3-Difluor-4-ethoxyphenyl)-trans-4-(trans-4-heptylcyclohexyl)-cyclohexanoat
(2,3-Difluor-4-methoxyphenyl)-trans-4-(trans-4-ethylcyclohexyl)-cyclohexanoat
(2,3-Difluor-4-methoxyphenyl)-trans-4-(trans-4-propylcyclohexyl)-cyclohexanoat
(2,3-Difluor-4-methoxyphenyl)-trans-4-(trans-4-butylcyclohexyl)-cyclohexanoat
(2,3-Difluor-4-methoxyphenyl)-trans-4-(trans-4-pentylcyclohexyl)-cyclohexanoat
(2,3-Difluor-4-methoxyphenyl)-trans-4-(trans-4-heptylcyclohexyl)-cyclohexanoat
(2,3-Difluor-4-propoxyphenyl)-trans-4-(trans-4-ethylcyclohexyl)-cyclohexanoat
(2,3-Difluor-4-propoxyphenyl)-trans-4-(trans-4-propylcyclohexyl)-cyclohexanoat
(2,3-Difluor-4-propoxyphenyl)-trans-4-(trans-4-butylcyclohexyl)-cyclohexanoat
(2,3-Difluor-4-propoxyphenyl)-trans-4-(trans-4-pentylcyclohexyl)-cyclohexanoat
(2,3-Difluor-4-propoxyphenyl)-trans-4-(trans-4-heptylcyclohexyl)-cyclohexanoat
(2,3-Difluor-4-butoxyphenyl)-trans-4-(trans-4-ethylcyclohexyl)cyclohexanoat
(2,3-Difluor-4-butoxyphenyl)-trans-4-(trans-4-propylcyclohexyl)-cyclohexanoat
(2,3-Difluor-4-butoxyphenyl)-trans-4-(trans-4-butylcyclohexyl)-cyclohexanoat
(2,3-Difluor-4-butoxyphenyl)-trans-4-(trans-4-pentylcyclohexyl)-cyclohexanoat
(2,3-Difluor-4-butoxyphenyl)-trans-4-(trans-4-heptylcyclohexyl)-cyclohexanoat

Beispiel 3

0,1 mol 4-Hexyloxybenzoesäure, 0,01 mol Dimethylaminopyridin und 0,1 mol 2,3-Difluor-4-octyloxyphenol (herstellbar aus 2,3-Difluoroctyloxybenzol durch Lithiierung bei -70 bis -80° und Zutropfen einer aus 0,12 mol t-Butylhydroperoxid und 0,12 mol Butyllithium bereiteten Lösung von Lithium-t-butylperoxid in Ether) werden in 150 ml Dichlormethan vorgelegt, bei 10° unter Rühren eine lösung von 0,1 mol Dicyclohexylcarbodiimid in 30 ml Dichlormethan zugetropft und anschließend 15 Stunden bei Raumtemperatur nachgerührt. Man filtriert über Kieselgel ab, verdampft das Lösungsmittel und erhält als Rückstand (2,3-Difluor-4-octyloxyphenyl)-4-hexyloxybenzoat.

Analog werden hergestellt:
(2,3-Difluor-4-octyloxyphenyl)-p-(4-heptyloxy-3-fluorphenyl)-benzoat, K 69,3° $S_C$ 146,9° $S_A$ 152,7° N 156° I
(2,3-Difluor-4-octyloxyphenyl)-p-(4-octyloxy-3-fluorphenyl)-benzoat

(2,3-Difluor-4-octyloxyphenyl)-p-(4-nonyloxy-3-fluorphenyl)-benzoat, K 71,7° $S_C$ 146° $S_A$ 149,8° N 150,2 I
(2,3-Difluor-4-octyloxyphenyl)-p-(4-decyloxy-3-fluorphenyl)-benzoat
(2,3-Difluor-4-octyloxyphenyl)-p-(4-heptyl-3-fluorphenyl)-benzoat, C 81° $S_C$ 121° $S_A$ 128,5° N 148° I
(4-Heptyloxy-2,3-difluorphenyl)-p-hexylbenzoat
(4-Heptyloxy-2,3-difluorphenyl)-p-heptylbenzoat
(4-Heptyloxy-2,3-difluorphenyl)-p-octylbenzoat, F 43,5°
(4-Heptyloxy-2,3-difluorphenyl)-p-nonylbenzoat
(4-Heptyloxy-2,3-difluorphenyl)-p-decylbenzoat
(4-Heptyloxy-2,3-difluorphenyl)-p-hexyloxybenzoat
(4-Heptyloxy-2,3-difluorphenyl)-p-heptyloxybenzoat
(4-Heptyloxy-2,3-difluorphenyl)-p-octyloxybenzoat, C 53° ($S_C$ 39°) N 57° I
(2,3-Difluor-4-heptyloxyphenyl)-p-(4-hexyl-3fluorphenyl)-benzoat
(2,3-Difluor-4-heptyloxyphenyl)-p-(4-heptyl-3fluorphenyl)-benzoat
(2,3-Difluor-4-heptyloxyphenyl)-p-(4-octyl-3fluorphenyl)-benzoat, C 60° $S_C$ 150° $S_A$ 155° N 157° I
(2,3-Difluor-4-heptyloxyphenyl)-p-(4-hexylphenyl)-benzoat
(2,3-Difluor-4-heptyloxyphenyl)-p-(4-heptylphenyl)-benzoat
(2,3-Difluor-4-heptyloxyphenyl)-p-(4-octylphenyl)-benzoat, C 86° $S_C$ 125° $S_A$ 131° N 145° I
(2,3-Difluor-4-heptyloxyphenyl)-p-(4-hexyloxyphenyl)-benzoat
(2,3-Difluor-4-heptyloxyphenyl)-p-(4-heptyloxyphenyl)-benzoat
(2,3-Difluor-4-heptyloxyphenyl)-p-(4-octyloxyphenyl)-benzoat, C 94° $S_C$ 157° $S_A$ 166° N 174° I
(4-Octyloxy-2,3-difluorphenyl)-p-hexylbenzoat
(4-Octyloxy-2,3-difluorphenyl)-p-heptylbenzoat
(4-Octyloxy-2,3-difluorphenyl)-p-octylbenzoat
(4-Octyloxy-2,3-difluorphenyl)-p-nonylbenzoat
(4-Octyloxy-2,3-difluorphenyl)-p-hexyloxybenzoat
(4-Octyloxy-2,3-difluorphenyl)-p-heptyloxybenzoat, K 45° N 53° I
(4-Octyloxy-2,3-difluorphenyl)-p-octyloxybenzoat, K 53° $S_C$ (39°) N 59,8° I
(4-Octyloxy-2,3-difluorphenyl)-p-nonyloxybenzoat, K 53,6° $S_C$ (49°) N 59,3° I
(4-Octyloxy-2,3-difluorphenyl)-trans-4-pentylcyclohexylcarboxylat, K 30° N 60° I
(4-Octyloxy-2,3-difluorphenyl)-trans,trans-4′-pentylbicyclohexyl-4-ylcarboxylat, K 58° $S_C$ (38°) $S_A$ 167° N 182,5° I
(4-Octyloxy-2,3-difluorphenyl)-4-(trans-4-pentylcyclohexyl)-benzoat, K 62° $S_C$ 63° $S_A$ 100° N 152,2° I
(2,3-Difluor-4-nonyloxyphenyl)-p-(4-heptyloxy-3-fluorphenyl)-benzoat
(2,3-Difluor-4-nonyloxyphenyl)-p-(4-octyloxy-3-fluorphenyl)-benzoat
(2,3-Difluor-4-nonyloxyphenyl)-p-(4-nonyloxy-3-fluorphenyl)-benzoat
(2,3-Difluor-4-nonyloxyphenyl)-p-(4-decyloxy-3-fluorphenyl)-benzoat
(2,3-Difluor-4-decyloxyphenyl)-p-(4-heptyloxy-3-fluorphenyl)-benzoat
(2,3-Difluor-4-decyloxyphenyl)-p-(4-octyloxy-3-fluorphenyl)-benzoat
(2,3-Difluor-4-decyloxyphenyl)-p-(4-nonyloxy-3-fluorphenyl)-benzoat
(2,3-Difluor-4-decyloxyphenyl)-p-(4-decyloxy-3-fluorphenyl)-benzoat

Beispiel 4

Man löst 1 mol 2,3-Difluorphenol in 2 l 10 %iger Natronlauge und tropft während 3-4 Stunden bei 20° eine gesättigte wäßrige Lösung von Kaliumperoxodisulfat zu. Nach dem Stehenlassen über Nacht säuert man die Lösung schwach an (pH 3-4) und extrahiert unumgesetztes Ausgangsmaterial mit Ether. Die wäßrige Phase wird mit Natriumhydrogencarbonat neutralisiert und im Vakuum zur Trockne eingeengt. Aus dem Rückstand löst man das 2,3-Difluor-4-hydroxyphenylkaliumsulfat mit 90%igem Ethanol heraus und dampft den Alkohol ab.

Zu 25 g Rohprodukts (~ 0,1 mol) werden in wäßrigalkoholischer Natronlauge (7 g NaOH in 100 ml 50%igem Alkohol) unter Rühren und Rückflußkochen 14 g Benzylchlorid zugetropft. Man läßt 2 Stunden nachreagieren, säuert mit Salzsäure schwach an und erhitzt weitere 2 Stunden. Nach dem Abkühlen wird das 4-Benzyloxy-2,3-difluorphenol extrahiert und nach Abziehen des Lösungsmittels destilliert.

Analog werden durch Umsetzung des 2,3-Difluor-4-hydroxyphenylkaliumsulfats mit Alkylbromiden und -sulfaten Monoalkylether des 2,3-Difluorhydrochinons erhalten.

0,1 mol 2,3-Difluor-4-benzyloxyphenol werden mit 0,1 mol Pyridin und 0,1 mol 4-Ethoxybenzoylchlorid in Toluol verestert, der entstehende Ester isoliert und anschließend der Benzylether hydrogenolytisch mit Palladiumkohle als Katalysator gespalten.

Durch Veresterung der freien Hydroxygruppe mit Buttersäure/Dicyclohexylcarbodiimid erhält man (2,3-Difluor-4-butyryloxyphenyl)-4-ethoxybenzoat.

Beispiel 5

Durch Veresterung des 2,3-Difluor-4-benzyloxyphenols mit 2-Fluor-4-(5-hexylpyrimidin-2-yl)-benzoesäure (hergestellt durch Verseifung des Nitrils über den Imidoester) und Dicyclohexylcarbodiimid, hydrierende Spaltung des Benzylethers und erneute Veresterung mit Octansäure analog Beispiel 4 erhält man (2,3-Difluor-4-octanoyloxyphenyl)-2-fluor-4-(5-hexylpyrimidin-2-yl)-benzoat.

Beispiel 6

0,1 mol 2,3-Difluor-4-(trans-4-pentylcyclohexyl)-methoxyphenol, 0,11 mol Butylbromid und 0,11 mol Kaliumcarbonat werden in 100 ml Dimethylformamid (DMF) 16 Stunden auf 100° erhitzt. Nach dem Abkühlen saugt man die anorganischen Salze ab, engt das Filtrat ein und versetzt mit Wasser. Durch Extraktion mit Dichlormethan wird 1-Butyloxy-2,3-difluor-4-(trans-4-pentylcyclohexyl)methoxybenzol erhalten.

Analog werden hergestellt:

1-Ethoxy-2,3-difluor-4-(trans-4-pentylcyclohexyl)-methoxy-benzol, K 38° N (O°) I
1-Ethoxy-2,3-difluor-4-[trans-4-(trans-4-propyl-cyclohexyl)-cyclohexyl] methoxy-benzol, K 63° N 153° I.
1-Ethoxy-2,3-difluor-4-[trans-4-(trans-4-butylcyclohexyl)-cyclohexyl]-methoxy-benzol
1-Ethoxy-2,3-difluor-4-[trans-4-(trans-4-pentylcyclohexyl)-cyclohexyl]-methoxy-benzol
1-Methoxy-2,3-difluor-4-[trans-4-(trans-4-ethylcyclohexyl)-cyclohexyl]-methoxy-benzol
1-Methoxy-2,3-difluor-4-[trans-4-(trans-4-propylcyclohexyl)-cyclohexyl]-methoxy-benzol
1-Methoxy-2,3-difluor-4-[trans-4-(trans-4-butylcyclohexyl)-cyclohexyl]-methoxy-benzol
1-Methoxy-2,3-difluor-4-[trans-4-(trans-4-pentylcyclohexyl)-cyclohexyl]-methoxy-benzol
1-Methoxy-2,3-difluor-4-[trans-4-(trans-4-heptylcyclohexyl)-cyclohexyl]-methoxy-benzol
1-Propoxy-2,3-difluor-4-[trans-4-(trans-4-ethylcyclohexyl)-cyclohexyl]-methoxy-benzol
1-Propoxy-2,3-difluor-4-[trans-4-(trans-4-propylcyclohexyl)-cyclohexyl]-methoxy-benzol
1-Propoxy-2,3-difluor-4-[trans-4-(trans-4-butylcyclohexyl)-cyclohexyl]-methoxy-benzol
1-Propoxy-2,3-difluor-4-[trans-4-(trans-4-pentylcyclohexyl)-cyclohexyl]-methoxy-benzol
1-Propoxy-2,3-difluor-4-[trans-4-(trans-4-heptylcyclohexyl)-cyclohexyl]-methoxy-benzol
1-Butoxy-2,3-difluor-4-[trans-4-(trans-4-ethylcyclohexyl)-cyclohexyl]-methoxy-benzol
1-Butoxy-2,3-difluor-4-[trans-4-(trans-4-propylcyclohexyl)-cyclohexyl]-methoxy-benzol
1-Butoxy-2,3-difluor-4-[trans-4-(trans-4-butylcyclohexyl)-cyclohexyl]-methoxy-benzol
1-Butoxy-2,3-difluor-4-[trans-4-(trans-4-pentylcyclohexyl)-cyclohexyl]-methoxy-benzol
1-Butoxy-2,3-difluor-4-[trans-4-(trans-4-heptylcyclohexyl)-cyclohexyl]-methoxy-benzol

Beispiel 7

Aus 0,1 mol 2,3-Difluor-4-(trans-4-pentylcyclohexyl)methoxyphenol, 0,1 mol trans-4-(trans-4-Propylcyclohexyl)cyclohexyl-methyljodid und 0,1 mol Kaliumcarbonat erhält man nach der Vorschrift von Beispiel 6 1-[trans-4-(trans-4-propylcyclohexyl)cyclohexylmethoxy]-2,3-di-fluor-4-(trans-4-pentylcyclohexylmethoxy)-benzol.

Beispiel 8

Man löst 1 mol 2,3-Difluorphenol in 1 l 10%iger Natronlauge und läßt innerhalb 4 Stunden unter Eiskühlung eine gesättigte wäßrige Lösung von 1,1 mol Kaliumperoxodisulfat zutropfen. Die Temperatur soll dabei nicht über 20° steigen. Man rührt über Nacht bei Raumtemperatur, neutralisiert mit Salzsäure und extrahiert unumgesetztes Ausgangsprodukt mit Ether. Die wäßrige Phase wird mit 3 l Toluol und 2 mol Natriumsulfit versetzt, mit 1 l konz. Salzsäure stark sauer gestellt und 20 Minuten unter Rückfluß erhitzt. Nach dem Abkühlen trennt man die Toluolschicht ab, extrahiert die wäßrige Phase mit Ether im Perforator und erhält durch Einengen der vereinigten Toluol- und Etherextrakte das 2,3-Difluorhydrochinon.

Beispiel 9

0,05 mol 2,3-Difluorhydrochinon und 0,1 mol Pyridin werden in 100 ml Toluol gelöst. Bei 80° tropft man 0,1 mol trans-4-Butylcyclohexancarbonsäurechlorid zu und rührt 3 Stunden nach. Die übliche Aufarbeitung

ergibt 2,3-Difluor-1,4-bis(trans-4-butylcyclohexylcarbonyloxy)-benzol.

Analog werden hergestellt:

2,3-Difluor-1,4-bis-(trans-4-propylcyclohexylcarbonyloxy)-benzol
2,3-Difluor-1,4-bis-(trans-4-pentylcyclohexylcarbonyloxy)-benzol, K 87° N 208° I
2,3-Difluor-1,4-bis-(trans-4-hexylcyclohexylcarbonyloxy)-benzol
2,3-Difluor-1,4-bis-(trans-4-heptylcyclohexylcarbonyloxy)-benzol
2,3-Difluor-1,4-bis-(trans-4-octylcyclohexylcarbonyloxy)-benzol, K 80° $S_F$ 94° $S_I$ 105° $S_C$ 124° N 176° I
2,3-Difluor-1,4-bis-(trans-4-nonylcyclohexylcarbonyloxy)-benzol
2,3-Difluor-1,4-bis-(trans-4-decylcyclohexylcarbonyloxy)-benzol

Beispiel 10

Analog Beispiel 4 erhält man durch Umsetzung von 2,3-Difluor-4-benzyloxyphenolmit trans-4-Propylcyclohexancarbonsäurechlorid/Pyridin, hydrogenolytische Spaltung des Benzylethers und erneute Veresterung mit trans-4-Pentylcyclohexancarbonsäure/Dicyclohexylcarbodiimid in Gegenwart von 4-Dimethylaminopyridin 2,3-Difluor-1-(trans-4-propylcyclohexanoyloxy)-4-(trans-4-pentylcyclohexanoyloxy)-benzol.

Die folgenden Beispiele betreffen flüssigkristalline Phasen mit mindestens einer erfindungsgemäßen Verbindung:

Beispiel A

Eine flüssigkristalline Phase bestehend aus

| | |
|---|---|
| 6 % | 4-Octyloxyphenyl-4-decyloxybenzoat, |
| 9 % | 4-Nonyloxyphenyl-4-decyloxybenzoat, |
| 14 % | 4-Decyloxyphenyl-4-decyloxybenzoat, |
| 5 % | 2,3-Difluor-4-octyloxyphenyl-4-octyloxybenzoat, |
| 7 % | 2,3-Difluor-4-octyloxyphenyl-4-decyloxybenzoat, |
| 9 % | 2,3-Difluor-4-decyloxyphenyl-4-decyloxybenzoat, |
| 4 % | 2,3-Difluor-4-nonanoyloxyphenyl-4-octyloxybenzoat, |
| 10 % | 4′-Pentyloxybiphenyl-4-yl-4-octyloxybenzoat, |
| 8 % | 4′-Heptyloxybiphenyl-4-yl-4-octyloxybenzoat, |
| 17 % | r-1-Cyan-cis-4-(4′-octyloxybiphenyl-4-yl)-1-octylcyclohexan und |
| 11 % | chilarem 2-Chlor-3-methylbuttersäure-[p-(5-heptylpyrimidin-2-yl)-phenylester] |

zeigt $S_c^*$ 62 $S_A$ 68 Ch 81 I und eine Spontanpolarisation von 13 $nC/cm^2$ bei Raumtemperatur.

Beispiel B

Eine flüssigkristalline Phase bestehend aus

| | |
|---|---|
| 8 % | 2-p-Octyloxyphenyl-5-octylpyrimidin, |
| 10 % | 2-p-Nonyloxyphenyl-5-octylpyrimidin, |
| 12 % | 2-p-Octyloxyphenyl-5-nonylpyrimidin, |
| 20 % | 2-p-Nonyloxyphenyl-5-nonylpyrimidin, |
| 7 % | 2,3-Difluor-4-decyloxyphenyl-4-decyloxybenzoat, |
| 7 % | 2,3-Difluor-4-octyloxyphenyl-4-decyloxybenzoat, |
| 9 % | 2-(p-Heptylphenyl)-5-(p-hexyloxyphenyl)-1,3,4-thiadiazol, |
| 9 % | 2-(p-Heptylphenyl-5-(p-octyloxyphenyl)-1,3,4-thiadiazol, |
| 8 % | 2,3-Difluor-4-octanoyloxyphenyl-4-(5-hexylpyrimidin-2-yl)-2-fluorbenzoat und |
| 10% | chiralem Isopropyl-2-[p-(p-decyloxyphenyl)-phenoxy]-propionat |

zeigt $S_c^*$ 58 $S_A$ und eine Spontanpolarisation von 11 $nC/cm^2$ bei Raumtemperatur.

Beispiel C

Eine nematische flüssigkristalline Phase bestehend aus

| | |
|---|---|
| 7 % | 2-p-Cyanphenyl-5-propyl-1,3-dioxan, |
| 7 % | 2-p-Cyanphenyl-5-butyl-1,3-dioxan, |
| 6 % | 2-p-Cyanphenyl-5-pentyl-1,3-dioxan, |
| 10 % | trans-1-p-Propylphenyl-4-pentylcyclohexan, |
| 8 % | 4-Cyan-4′-(trans-4-pentylcyclohexyl)-biphenyl, |
| 6 % | 4-Ethyl-4′-(trans-4-pentylcyclohexyl)-biphenyl, |
| 4 % | 4-Ethyl-4′-(trans-4-propylcyclohexyl)-biphenyl, |
| 5 % | 2-p-Pentyloxyphenyl-5-hexylpyrimidin, |
| 6 % | 2-p-Heptyloxyphenyl-5-hexylpyrimidin, |
| 5 % | 2-p-Methoxyphenyl-5-hexylpyrimidin, |
| 8 % | 2-p-Heptyloxyphenyl-5-hexylpyrimidin, |
| 8 % | 2-p-Nonyloxyphenyl-5-hexylpyrimidin, |
| 4 % | trans-4-Propylcyclohexancarbonsäure-(p-methoxyphenylester), |
| 7 % | (2,3-Difluor-4-ethoxyphenyl)-trans-4-pentylcyclohexanoat, |
| 5 % | (2,3-Difluor-4-ethoxyphenyl)-trans-4-(trans-4-propylcyclohexyl)-cyclohexanoat und |
| 4% | Buttersäure-(p-trans-4-propylcyclohexylphenylester) |

zeichnet sich durch gute Multiplexeigenschaften aus.

Beispiel D

Eine flüssigkristalline Phase bestehend aus

| | |
|---|---|
| 12,5 % | 4-Heptyloxy-2,3-difluorphenyl 4′-octyloxybiphenyl-4-ylcarboxylat |
| 14,2 % | 4-Heptyloxy-2,3-difluorphenyl 4-octyloxybenzoat |
| 12,5 % | 4-Heptyl-2-fluorphenyl 4′-heptyloxybiphenyl-4-ylcarboxylat |
| 12,5 % | 4-Heptyl-2-fluorphenyl 4′-heptyloxy-2′-fluorbiphenyl-4-ylcarboxylat |
| 14,2 % | 4-Octyloxy-3-fluorphenyl 4-octyloxybenzoat |
| 12,34 % | 4-Pentyl-2-fluorphenyl 4-octyloxybenzoat |
| 14,24 % | 4-Octyloxy-3-fluorphenyl 4-heptyloxybenzoat |
| 5,04 % | chiralem 4-(2-Methylbutyl)-phenyl 4′-octylbiphenyl-4-ylcarboxylat und |
| 2,48 % | chiralem 1-Cyano-2-methylpropyl 4′-octyloxybiphenyl-4-ylcarboxylat |

zeigt $S_C^*$ 66,4° $S_A$ 73° Ch 97,2° I und eine Spontanpolarisation von 9 nC/cm$^2$ bei 30 °C.

Beispiel E

Eine flüssigkristalline Phase bestehend aus

| | |
|---|---|
| 16,87 % | 4-Heptyl-2-fluorphenyl 4′-heptyloxybiphenyl-4-ylcarboxylat |
| 16,87 % | 4-Heptyl-2-fluorphenyl 4′-heptyloxy-2′-fluorbiphenyl-4-ylcarboxylat-4- |
| 16,87 % | 4-Octyl-2-fluorphenyl 4′-octyloxy-2′,3′-difluorbiphenyl-4-ylcarboxylat |
| 14 % | 4-Octyloxy-3-fluorphenyl 4-octyloxybenzoat |
| 14 % | 4-Hexyloxy-3-fluorphenyl 4-octyloxybenzoat |
| 10 % | 4-Octyloxy-2-fluorphenyl 4-pentylbenzoat |
| 9 % | 4-Heptyl-2-fluorphenyl 4′-octyloxy-2′,3′-difluorbiphenyl-4-ylcarboxylat und |
| 2,4 % | chiralem 1-Cyano-2-methylpropyl 4′-octyloxybiphenyl-4-ylcarboxylat |

zeigt $S_C^*$ 71,8° $S_A$ 81° Ch 103,8° I und eine hohe Spontanpolarisation.

**Patentansprüche**

**1.** Derivate des 2,3-Difluorhydrochinons der Formel I,

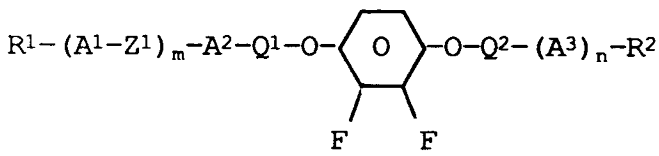

I

worin

$R^1$ und $R^2$ jeweils unabhängig voneinander unsubstituiertes, einfach durch Cyan oder mindestens einfach durch Fluor oder Chlor substituiertes Alkyl mit 1 bis 15 C-Atomen oder Alkenyl mit 3 bis 15 C-Atomen, wobei in diesen Resten auch eine $CH_2$-Gruppe durch -O-, -CO-, -CO-O-, -O-CO- oder -O-CO-O- ersetzt sein kann,

$A^1$ , $A^2$ und $A^3$ jeweils unabhängig voneinander

(a) trans-1,4-Cyclohexylen, worin auch eine oder zwei nicht benachbarte $CH_2$-Gruppen durch -O- und/oder -S- ersetzt sein können,

(b) 1,4-Phenylen, worin auch eine oder zwei CH-Gruppen durch N ersetzt sein können,

(c) Rest aus der Gruppe bestehend aus Piperidin-1,4-diyl, 1,4-Bicyclo(2,2,2)-octylen-, 1,3,4-Thiadiazol-2,5-diyl, Naphthalin,-2,6-diyl-, Decahydronaphthalin-2,6-diyl- und 1,2,3,4-Tetrahydronaphthalin-2,6-diyl,

wobei die Reste (a) und (b) durch F, Cl, $CH_3$ oder CN ein-oder mehrfach substituiert sein können,

$Z^1$ -CO-O, -O-CO, $-CH_2-CH_2-$, $-OCH_2-$, $-CH_2O-$, -C≡C- oder eine Einfachbindung,

$Q^1$ und $Q^2$ jeweils unabhängig voneinander -CO- oder $-CH_2-$,

m und n jeweils 0, 1 oder 2, und

(m + n) 0, 1 oder 2 bedeutet.

**2.** Derivate des 2,3-Difluorhydrochinons der Formel Ia',

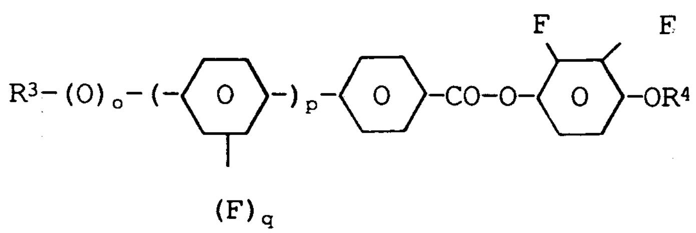

Ia'

worin

R und $R^4$ jeweils unabhängig voneinander Alkyl mit 1 bis 15 C-Atomen, und o, p und q 0 oder 1 bedeutet.

**3.** Derivate des 2,3-Difluorhydrochinons nach Anspruch 1, dadurch gekennzeichnet, daß sie der Formel Ia'' entsprechen,

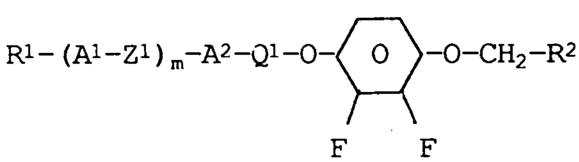

Ia''

worin m 0 oder 1, $Z^1$ $-CH_2CH_2-$ oder eine Einfachbindung, und $A^1$ und $A^2$ jeweils trans-1,4-Cyclohexylen bedeuten.

**4.** Verfahren zur Herstellung der Derivate des 2,3-Difluorhydrochinons nach Anspruch 1, dadurch gekennzeichnet, daß man eine Hydroxyverbindung der Formel

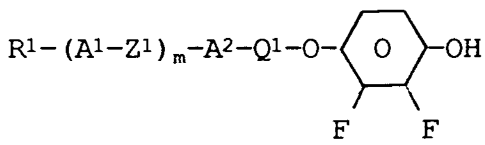

oder eines ihrer reaktionsfähigen Derivate mit einer Verbindung der Formel

$R^2$-$(A^3)_n$-$Q^2$-OH

oder einem ihrer reaktionsfähigen Derivate verestert ($Q^2$ = CO) oder verethert ($Q^2$ = $CH_2$), oder daß man eine Hydroxyverbindung der Formel

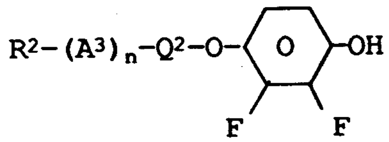

oder eines ihrer reaktionsfähigen Derivate mit einer Verbindung der Formel

$R^1$-$(A^1$-$Z^1)_m$-$A^2$-$Q^1$-OH

oder einem ihrer reaktionsfähigen Derivate verestert ($Q^1$ = CO) oder verethert ($Q^1$ = $CH_2$).

**5.** Verwendung der Derivate nach Anspruch 1 als Komponenten flüssigkristalliner Medien.

**6.** Flüssigkristallines Medium mit mindestens zwei flüssigkristallinen Komponenten, dadurch gekennzeichnet, daß mindestens eine Komponente ein Derivat nach Anspruch 1 ist.

**7.** Elektrooptisches Anzeigeelement, dadurch gekennzeichnet, daß es als Dielektrikum ein Medium nach Anspruch 6 enthält.

## Claims

**1.** 2,3-Difluorohydroquinone derivatives of the formula I,

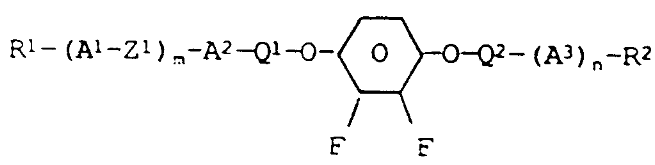

I

in which

$R^1$ and $R^2$, in each case independently of one another, are alkyl having 1 to 15 C atoms or alkenyl having 3 to 15 C atoms which are unsubstituted, monosubstituted by cyano or at least monosubstituted by fluorine or chlorine, it also being possible for one $CH_2$ group in these radicals to be replaced by -O-, -CO-, -CO-O-, -O-CO- or -O-CO-O-,

$A^1$, $A^2$ and $A^3$, in each case independently of one another, are

(a) trans-1,4-cyclohexylene, in which, in addition, one or two non-adjacent $CH_2$ groups may be replaced by -O- and/or -S-,

(b) 1,4-phenylene, in which, in addition, one or two CH groups may be replaced by N, or

(c) a radical from the group comprising piperidine-1,4-diyl, 1,4-bicyclo(2,2,2)-octylene-[sic], 1,3,4-thiadiazole-2,5-diyl, naphthalene-2,6-diyl-[sic], decahydronaphthalene-2,6-diyl-[sic] and 1,2,3,4-tetrahydronaphthalene-2,6-diyl,

it being possible for the radicals (a) and (b) to be monosubstituted or polysubstituted by F, Cl, $CH_3$ or CN,

| | |
|---|---|
| $Z^1$ | is -CO-O-, -O-CO-, $-CH_2-CH_2-$, $-OCH_2-$, $-CH_2O-$, -C≡C- or a single bond, |
| $Q^1$ and $Q^2$, | in each case independently of one another, are -CO- or $-CH_2-$, |
| m and n | are each 0, 1 or 2, and |
| (m + n) | is 0, 1 or 2. |

**2.** 2,3-Difluorohydroquinone derivatives of the formula Ia'

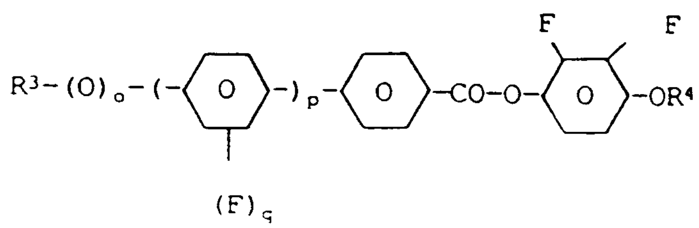

Ia'

in which

$R^3$ and $R^4$, in each case independently of one another, are alkyl having 1 to 15 C atoms and o, p and q are 0 or 1.

**3.** 2,3-Difluorohydroquinone derivatives according to Claim 1, characterised in that they correspond to the formula Ia"

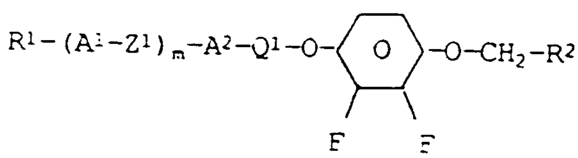

Ia''

in which m is 0 or 1, $Z^1$ is $-CH_2CH_2-$ or a single bond, and $A^1$ and $A^2$ are each trans-1,4-cyclohexylene.

**4.** Process for the preparation of 2,3-difluorohydroquinone derivatives according to Claim 1, characterized in that a hydroxyl compound of the formula

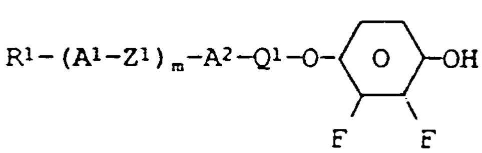

or a reactive derivative thereof is esterified ($Q^2$ = CO) or etherified ($Q^2$ = $CH_2$) using a compound of the formula

$R^2-(A^3)_n-Q^2-OH$

or a reactive derivative thereof, or in that a hydroxyl compound of formula

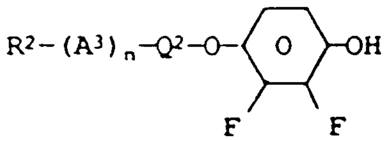

or a reactive derivative thereof is esterified ($Q^1$ = CO) or etherified ($Q^1$ = $CH_2$) using a compound of the formula

$R^1$-($A^1$-$Z^1$)$_m$-$A^2Q^1$-OH

or a reactive derivative thereof.

**5.** Use of the derivatives according to Claim 1 as components of liquid-crystalline media.

**6.** Liquid-crystalline medium containing at least two liquid-crystalline components, characterized in that at least one component is a derivative according to Claim 1.

**7.** Electrooptical display element, characterized in that it contains, as dielectric, a medium according to Claim 6.

**Revendications**

**1.** Dérivés de la 2,3-difluorhydroquinone répondant à la formule I

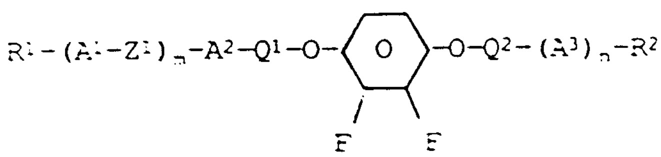

I

dans laquelle

$R^1$ et $R^2$ désignent chacun indépendamment l'un de l'autre un groupe alkyle en $C_1$ et $C_{15}$ ou alcényle en $C_3$ et $C_{15}$ non substitué, substitué une fois par un groupe cyano ou au moins une fois par un atome de fluor ou de chlore, un groupe $CH_2$ pouvant être également sustitué par un groupe -O-, -CO-, -CO-O-, -O-CO ou -O-CO-O-, dans ses radicaux,

$A^1$, $A^2$ et $A^3$ désignent chacun indépendamment les uns des autres

(a) un groupe trans-1,4-cyclohexylène, dans lequel un ou deux groupes $CH_2$ non voisins vont également être remplacés par -O- et/ou -S-,

(b) un groupe 1,4-phénylène, dans lequel un ou deux groupes CH peuvent également être substitués par N,

(c) le radical choisi parmi les groupes pipéridine-1,4-diyle, 1,4-bicylo(2,2,2) octylène, 1,3,4-thiadiazol-2,5-diyle, naphtalène-2,6-diyle, décahydronaphtalène-2,6-diyle et 1,2,3,4-tétrahydronaphtalène-2,6-diyle,

les radicaux (a) et (b) pouvant être substitués une ou plusieurs fois par F, Cl, $CH_3$ ou CN

$Z^1$ désigne les groupes -CO-O-, -O-CO, $-CH_2-CH_2-$, - $OCH_2$-, $-CH_2O$-, -C≡C- ou une simple liaison,

$Q^1$ et $Q^2$ désignent chacun indépendamment l'un de l'autre les groupes -CO- ou $-CH_2$-,

m et n désignent chacun 0, 1 ou 2 et

(m + n) est égal à 0,1 ou 2.

**2.** Dérivés de la 2,3-difluorhydroquinone répondant à la formule Ia'

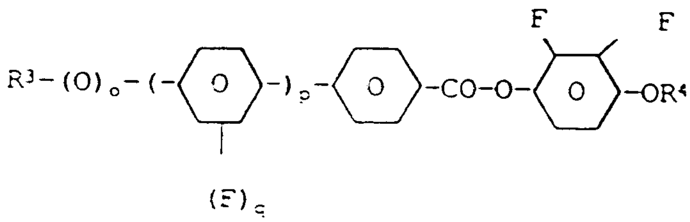

Ia'

dans laquelle

$R^3$ et $R^4$ désignent indépendamment l'un de l'autre un groupe alkyle en $C_1$ à $C_{15}$ et o, p et q sont égaux à 0 ou 1.

**3.** Dérivés de la 2,3-difluorhydroquinone selon la revendication 1, caractérisés en ce qu'ils répondent à la formule Ia",

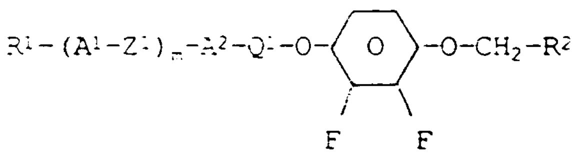

Ia''

dans laquelle m est égal à 0 ou 1, $Z^1$ est un groupe - $CH_2CH_2$- ou une simple liaison, et $A^1$ et $A^2$ désignent chacun un groupe trans-1,4-cyclohexylène.

**4.** Procédé pour la préparation des dérivés de la 2,3-difluorhydroquinone selon la revendication 1, caractérisé en ce qu'on estérifie ($Q^2$ = CO) ou en ce qu'on éthérifie ($Q^2$ = $CH_2$) un composé hydroxylé répondant à la formule

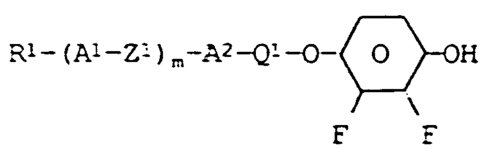

ou un de ses dérivés réactifs avec un composé répondant à la formule

$R^2$-$(A^3)_n$-$Q^2$-OH

ou un de ses dérivés réactifs, ou en ce qu'on estérifie ($Q^1$ = CO) ou en ce qu'on éthérifie ($Q^1$ = $CH_2$) un composé hydroxylé répondant à la formule

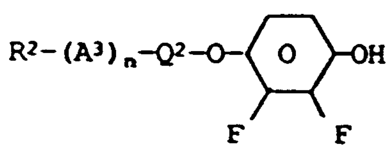

ou un de ses dérivés réactifs avec un composé répondant à la formule

$R^1$-$(A^1$-$Z^1)_m$-$A^2$-$Q^1$-OH

ou un de ses dérivés réactifs.

**5.** Utilisation des dérivés selon la revendication 1, comme constituants de milieux à cristaux liquides.

6. Milieu à cristaux liquides ayant au moins deux constituants à cristaux liquides, caractérisé en ce qu'au moins un constituant est un dérivé selon la revendication 1.

7. Elémént d'affichage électro-optique, caractérisé en ce qu'il contient comme diélectrique un milieu selon la revendication 6.